# EUROPEAN PATENT APPLICATION

(11) **EP 3 434 252 A1**
(43) Date of publication of application: **30.01.2019**
(21) Application number: 17770271.9
(22) Date of filing: 22.03.2017
(51) Int. Cl.: A61J 1/05, A61M 5/28, A61M 5/32

(54) **RUBBER STOPPER FOR DRUG CONTAINER, DRUG-ACCOMMODATING DRUG CONTAINER, AND DRUG CONTAINER SET**

(30) Priority: 23.03.2016 JP 2016059207
(71) Applicant: Terumo Kabushiki Kaisha, Tokyo 151-0072 (JP)
(72) Inventor: IIBUCHI, Ruriko, Ashigarakami-gun Kanagawa 259-0151 (JP); KAWAMURA, Masao, Ashigarakami-gun Kanagawa 259-0151 (JP); ARINOBE, Manabu, Ashigarakami-gun Kanagawa 259-0151 (JP)
(74) Representative: Casalonga
(86) International application number: PCT/JP2017/011419
(87) International publication number: WO 2017/164231

(57) **Abstract**

A rubber stopper 1 for a drug container is used for a drug container 2 having a main body and a neck portion having a diameter smaller than the main body. The rubber stopper 1 includes a main body 3, and a container insertion portion 4 extending downward from the main body 3 and capable of fitting into an opening portion of the container 2. The container insertion portion includes an outer surface portion 40 which comes into contact with an inner surface of the neck portion of the container when attached to the container 2, an upper inner portion which forms a columnar gap portion 45 extending in a substantially uniform shape downward from a central portion of a lower surface of the main body 3, and inclined surfaces 61 and 62 extending obliquely downward toward the outer surface portion 40 of the container insertion portion 4 from the lower end of the upper inner surface portion. The upper end portion 48 of the outer surface portion annularly and liquid-tightly comes into contact with the inner surface of the neck portion of the container, and the lower ends of the inclined surfaces reach or approach the outer surface portion of the container insertion portion.

## Description

### Technical Field

The present invention relates to a drug-container rubber stopper, a drug-accommodating drug container using the same, and a drug container set using the drug-accommodating drug container.

### Background Art

Conventionally, many medical containers in which drugs are accommodated in a container sealed with a rubber stopper are provided. As a drug container, a so-called vial bottle is generally used. Examples of the drug to be accommodated in the vial bottle include powders (for example, freeze-dried preparations and powdered preparations), liquid preparations, and the like. In addition, at the time of administration of the drug, a drug solution for administration is usually prepared by injecting a medical liquid such as a dissolved liquid or diluted liquid into the vial bottle. However, in some cases, foaming occurs at the time of injection of the medical liquid. In particular, depending on the composition including the surfactant or the like, there are some powders which are easily foamed, and once foaming occurs, it is required to wait for administration until the foaming disappears.

In recent years, a resin hollow needle which cannot be punctured by a human is used for injecting the medical liquid into the vial bottle, instead of a so-called injection needle. The resin hollow needle generally has a closed puncture end and a side hole. Even when such a side hole type hollow needle is used, since the medical liquid discharged from the side hole drops downward as it is or hits the rubber stopper and drops downward, in some cases, the foaming occurs.

As a drug-container rubber stopper, there is, for example, one disclosed in JP 2006-110333 A (Patent Literature 1). The medicine container set includes a combination of a container made up of a cylindrical drug solution accommodating portion (1) and a drug solution injection port portion (2), and a rubber stopper (3) for sealing the port portion (2). The rubber stopper (3) includes a disc-shaped top surface portion (9) and a substantially cylindrical leg portion (10) provided on a lower surface (8) of the top surface portion. In the container, a flat surface (4) for bringing a lower end surface (7) of the leg portion (10) of the rubber stopper (3) into close contact with a boundary of an inner wall side between the drug solution accommodating portion (1) and the drug solution injection port portion (2), at least the side wall on the side of the drug solution accommodating portion (1) on which the drug solution is stored is formed with a rounded surface without corners, and when the drug solution injection port portion (2) is sealed with the rubber stopper (3), in a state in which an inner peripheral edge (5) of the lower end surface (7) of the leg portion (10) does not protrude to the inner side of the container than an inner peripheral edge (6) of the flat surface (4) of the container, the lower end surface (7) and the flat surface (4) are brought into close contact with each other.

Also, a drug-container rubber stopper is also disclosed in JP 2005-192888 A (Patent Literature 2).

### Citation List

### Patent Literature

Patent Literature 1: JP 2006-110333 A (USP 7934613, EP 1634819 A)
Patent Literature 2: JP 2005-192888 A

### Summary of Invention

### Technical Problem

In JP 2006-110333 A, there is no step between the inner surface of the rubber stopper and the inner surface of the container, and in the case of using a hollow needle having a side hole, when the liquid discharged from the side hole hits the rubber stopper, there is a possibility that a liquid flow may flow from the inner side surface of the rubber stopper to the inner surface of the container. However, in this case, since a step is not formed between the inner side surface of the rubber stopper and the inner surface of the container, it is necessary to provide a step having the flat surface (4) at the neck portion of the container.

For this reason, the formation of the container becomes complicated, or in the case of a container made of glass, the step itself cannot be molded well. Further, in a case where the rubber stopper disclosed in Patent Literature 1 is used for a normal drug container having no step in the neck portion, since the distal end of the rubber stopper has a predetermined thickness, even if the liquid discharged from the side hole hits the rubber stopper, there is a high possibility that the injected liquid drops from the distal end of the rubber stopper to the bottom of the container, thereby causing foaming. This also applies to the case of Patent Literature 2.

An object of the present invention is to provide a rubber stopper for drug container capable of allowing injected liquid to flow downward along the inner surface of the container without dropping directly into the container, when using a normal drug container having no step in the neck portion and a hollow needle having a side hole, a drug-accommodating drug container using the same, and a drug container set.

### Solution to Problem

Those which achieve the above object are as follows.

A drug-container rubber stopper configured to be attached to a drug container that comprises: a cylindrical body portion including a closed lower end and accommodating a drug therein, and a cylindrical neck portion extending upward from an upper end of the body portion and including an opening portion formed at an upper end thereof, an inner diameter of the neck portion being smaller than an inner diameter of the body portion and being substantially constant, the drug-container rubber stopper comprising: a main body; and a cylindrical container insertion portion extending downward from a lower surface of the main body and configured to fit into the neck portion of the drug container, wherein the main body includes a peripheral edge portion that is disposed on a radially outer side of the container insertion portion and that is configured to abut on an upper surface of the opening portion when attached to the drug container, wherein the container insertion portion comprises: an outer surface portion that is configured to come into contact with an inner surface of the neck portion of the drug container when attached to the drug container; an upper inner surface portion that forms a columnar gap portion extending downward in a substantially uniform shape from a central portion of the lower surface of the main body; and an inclined surface extending obliquely downward toward the outer surface portion of the container insertion portion from a lower end of the upper inner surface portion, at least an upper end portion of the outer surface portion being configured to annularly and liquid-tightly comes into contact with the inner surface of the neck portion, and a lower end of the inclined surface reaches or is close to the outer surface portion of the container insertion portion.

In addition, those that achieve the above object are as follows.

A drug-accommodating drug container comprising: the drug container; the drug-container rubber stopper that is attached to the drug container and seals the opening portion; and a drug accommodated in the drug container.

In addition, those that achieve the above object are as follows.

A drug container set including: the drug-accommodating drug container; and a liquid injection needle configured to pierce into the rubber stopper of the drug-accommodating drug container, in which the liquid injection needle includes a closed puncture end and a liquid discharging side hole disposed on a side portion of a front end portion thereof.

### Brief Description of Drawings

Fig. 1 is a front view of a drug-container rubber stopper according to an embodiment of the present invention.
Fig. 2 is a plan view of the drug-container rubber stopper illustrated in Fig. 1.
Fig. 3 is a bottom view of the drug-container rubber stopper illustrated in Fig. 1.
Fig. 4 is a right side view of the drug-container rubber stopper illustrated in Fig. 1.
Fig. 5 is a cross-sectional view taken along line A-A of Fig. 1.
Fig. 6 is a cross-sectional view taken along line B-B of Fig. 2.
Fig. 7 is a perspective view of the drug-container rubber stopper illustrated in Fig. 1 when viewed obliquely from below.
Fig. 8 is a perspective view of the drug-container rubber stopper illustrated in Fig. 1 when viewed obliquely from above.
Fig. 9 is a front view of the drug-container rubber stopper according to another embodiment of the present invention.
Fig. 10 is a plan view of the drug-container rubber stopper illustrated in Fig. 9.
Fig. 11 is a bottom view of the drug-container rubber stopper illustrated in Fig. 9.
Fig. 12 is a cross-sectional view taken along line C-C of Fig. 9.
Fig. 13 is a front view of a drug-accommodating drug container according to an embodiment of the present invention.
Fig. 14 is a plan view of the drug-accommodating drug container illustrated in Fig. 13.
Fig. 15 is a cross-sectional view taken along line D-D of Fig. 13.
Fig. 16 is a cross-sectional view taken along line E-E of Fig. 14.
Fig. 17 is an explanatory view illustrating a manufacturing process of the drug-accommodating drug container of the present invention.
Fig. 18 is an explanatory view illustrating the manufacturing process of the drug-accommodating drug container of the present invention.
Fig. 19 is a front view of a drug container set according to an embodiment of the present invention.
Fig. 20 is a front view of the liquid injection needle used in the drug container set illustrated in Fig. 19.
Fig. 21 is a cross-sectional view taken along line F-F of Fig. 20.
Fig. 22 is a front view of a drug container set according to another embodiment of the present invention.
Fig. 23 is an explanatory view illustrating the operation of the drug-container rubber stopper and the drug-accommodating drug container of the present invention.

### Description of Embodiments

A drug-container rubber stopper of the present invention will be described with reference to the embodiments illustrated in the drawings. A rubber stopper 1 for a drug container of the present invention is attached to a drug container 2. The drug container 2 has a cylindrical body portion 21 having a closed lower end and accommodating a drug therein, and a cylindrical neck portion 23 extending upward from an upper end of the body portion 21 and having an opening portion formed at an upper end thereof, and an inner diameter of the neck portion 23 is smaller than an inner diameter of the body portion 21 and is substantially constant.

Further, the rubber stopper 1 for the drug container includes a main body 3, and a cylindrical container insertion portion 4 extending downward from a lower surface of the main body 3 and capable of fitting into the neck portion 23 of the drug container 2. Further, the main body 3 has a peripheral edge portion which is disposed on a radially outer side of the container insertion portion 4 and abuts on the upper surface of the opening portion when attached to the drug container 2.

When attached to the drug container 2, the container insertion portion 4 has an outer surface portion 40 which comes into contact with an inner surface of the neck portion 23 of the drug container 2, an upper inner surface portion which forms a columnar gap portion 45 extending in a substantially uniform shape downward from a central portion of the lower surface of the main body 3, and inclined surfaces 61 and 62 extending obliquely downward toward the outer surface portion 40 of the container insertion portion 4 from a lower end of the upper inner surface portion. At least the upper end portion 48 of the outer surface portion 40 annularly and liquid-tightly comes into contact with the inner surface of the neck portion, and lower ends of the inclined surfaces 61 and 62 reach or are close to the outer surface portion 40 of the container insertion portion 4.

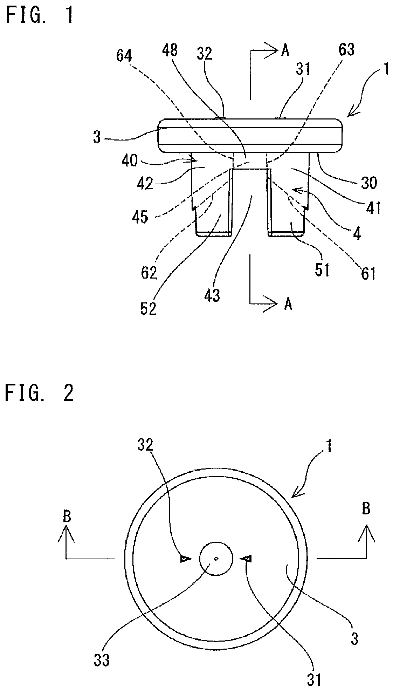

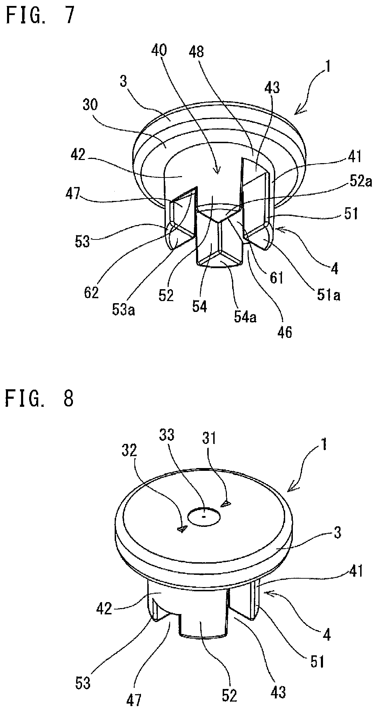

As illustrated in Figs. 1 to 8, the rubber stopper 1 for the drug container includes the disc-shaped main body 3, and the container insertion portion 4 extending downward from a center of the lower surface of the main body 3 and has an outer diameter smaller than the outer diameter of the main body.

In this embodiment, the main body 3 has a disc shape having a predetermined thickness. A lower surface peripheral edge portion 30 of the main body 3 constitutes an annular contact portion which makes contact with the upper surface of an opening portion 22 of the drug container 2 to be described later. The main body 3 is preferably in the shape of a disc. However, as long as the main body 3 has a peripheral edge portion which is disposed on the radially outer side of the container insertion portion 4 and abuts on the upper surface of the opening portion when attached to the drug container 2, it may not have a disc shape. For example, the main body 3 may have a polygonal plate-like shape having a peripheral edge portion disposed on the radially outer side of the container insertion portion 4. Further, the lower surface of the peripheral edge portion constitutes the lower surface peripheral edge portion 30.

The container insertion portion 4 has an arcuate outer surface or a columnar outer surface, and extends downward in substantially the same outer surface form. Further, a diameter of an imaginary circle that can be formed from the outer edge of a lower portion (the below-described leg part) of the container insertion portion 4 is smaller than that of the upper portion and the central part, which makes insertion into the drug container easier. Particularly, in the rubber stopper 1 of this embodiment, a diameter (or the diameter of the imaginary circle that can be formed from the outer edge) of the container insertion portion 4 gradually decreases from the upper end toward the lower end. In addition, the diameter of the upper portion of the container insertion portion 4 (or the diameter of the imaginary circle that can be formed from the outer edge) does not substantially change and may extend at the same diameter.

The outer surface portion 40 of the container insertion portion 4 makes contact with the inner peripheral surface of the opening portion 22 of the drug container 2 and forms a liquid-tight state. The upper portion of the container insertion portion 4 is strongly compressed by the inner peripheral surface of the opening portion 22 of the drug container 2. At least the upper end portion 48 of the outer surface portion 40 annularly and liquid-tightly makes contact with the inner surface of the neck portion 23 of the drug container 2. Therefore, the upper end portion 48 of the outer surface portion 40 has a columnar shape extending substantially at the same outer diameter. It is preferable that the inner diameter also extends substantially at the same inner diameter. Further, an axial length of the upper end portion 48 is preferably 1.0 to 4.0 mm, particularly preferably 2.0 to 3.0 mm.

As a constituent material of the rubber stopper 1, it is preferable to use an elastic material. Although there are no particular restrictions on the elastic material, it is possible to adopt various rubber materials (particularly vulcanized materials) such as natural rubber, isoprene rubber, butyl rubber, chloroprene rubber, nitrile-butadiene rubber, styrene-butadiene rubber, and silicone rubber. In particular, a diene type rubber is preferable from the viewpoints of elastic properties and capability of γ-ray sterilization, electron beam sterilization, and autoclave sterilization.

In the rubber stopper 1 for the drug container of this embodiment, the container insertion portion 4 has two facing columnar portions 41 and 42, and a columnar gap portion 45 located between the two columnar portions 41 and 42 and extending in a substantially uniform shape downward from the center upper portion (center) of the container insertion portion 4. The columnar gap portion 45 preferably extends in the uniform shape and size as in the illustrated embodiment, but it may be slightly larger downward.

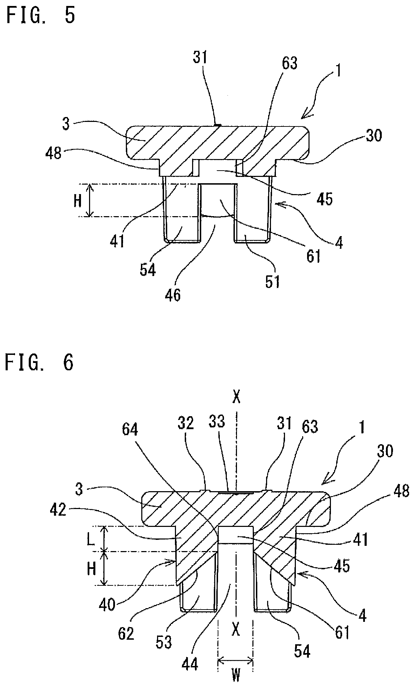

Each of the columnar portions 41 and 42 is provided with inclined surfaces 61 and 62 extending in a predetermined width toward the outer surface portion 40 of the container insertion portion 4 and obliquely downward with a part of the columnar gap portion 45 as a starting end (upper end). As illustrated in Fig. 6, two inclined surfaces 61 and 62 are provided to face each other over axis X of the columnar gap portion 45. Further, the lower ends of the inclined surfaces 61 and 62 reach or are close to the outer surface portion 40 of the container insertion portion 4. In this embodiment, as illustrated in Fig. 6, a junction between the lower ends of the inclined surfaces 61 and 62 and the outer surface portion 40 is integrated, and the junction is chamfered to have a rounded shape.

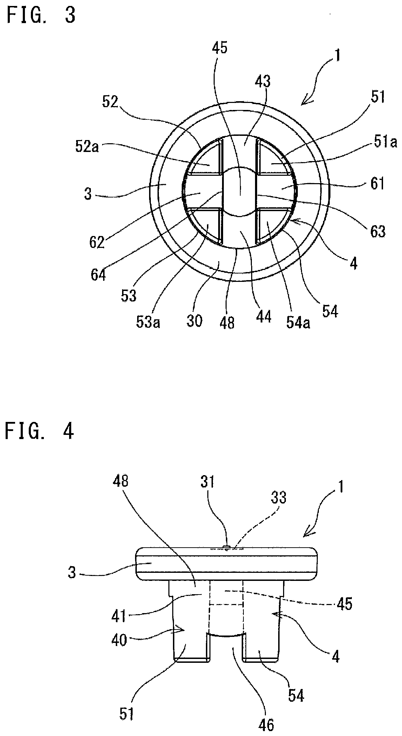

As illustrated in Fig. 6, the upper ends of the inclined surfaces 61 and 62 are located at a predetermined length lower than an inner upper end of the container insertion portion 4 (the columnar portions 41 and 42). Therefore, the columnar gap portion 45 maintains a columnar shape until it reaches the upper ends of the inclined surfaces 61 and 62. The container insertion portion 4 (the columnar portions 41 and 42) has facing inner surface portions 63 and 64 extending upward from the upper ends of the inclined surfaces 61 and 62. In other words, facing inner surface portions 63 and 64 are formed between the inner upper end of the container insertion portion 4 (the columnar portions 41 and 42) and the upper ends of the inclined surfaces 61 and 62. In the rubber stopper 1 for the drug container of this embodiment, as illustrated in Fig. 3, the inner surface portions 63 and 64 are flat plate-like inner walls. Further, the inner surface portions 63 and 64 may have a circular arc shape.

Further, as illustrated in Fig. 6, an axial length (L) from the upper end to the lower end of the columnar gap portion 45 formed by the inner surface portions 63 and 64, in other words, the distance between the inner upper end (lower surface) of the container insertion portion 4 (the columnar portions 41 and 42) and the upper ends of the inclined surfaces 61 and 62, is preferably 1 to 8 mm, particularly preferably 3 to 5 mm.

Further, the distance (W) between the facing inner surface portions 63 and 64, in other words, the width of the columnar gap portion 45 is preferably 1. 6 to 7 mm, particularly preferably 2.0 to 5.5 mm.

Further, in the rubber stopper 1 for the drug container of this embodiment, as illustrated in Fig. 3, the inclined surfaces 61 and 62 extend substantially in the same width from the upper end (start end) to the lower end (terminal end). Further, the inclined surfaces 61 and 62 may have a narrow width toward the lower end (terminal end), or may have a wider width. The width of the upper ends of the inclined surfaces 61 and 62 is preferably 1.8 to 5 mm, particularly preferably 2.5 to 4.0 mm. Furthermore, it is preferable that the lower ends of the inclined surfaces 61 and 62 do not exceed the lower end of the neck portion 23 of the drug container 2 when attached to the drug container 2.

In particular, it is preferable that the lower ends of the inclined surfaces 61 and 62 are located at a position higher by 0.5 mm or more than the lower end of the neck portion 23 of the drug container 2 when attached to the drug container 2. Further, the inclination angle of the inclined surfaces 61 and 62 (the inclination angle with respect to the central axis of the container insertion portion 4) is preferably 30 to 60 degrees, particularly preferably 35 to 45 degrees. Further, the width of the inclined surfaces 61 and 62 is preferably 1.5 to 8.0 mm, particularly preferably 2.0 to 6.0 mm.

Further, in the rubber stopper 1 for the drug container of this embodiment, as illustrated in Figs. 1 to 8, the container insertion portion 4 has leg portions 51, 52, 53, and 54 provided on both side portions of the inclined surfaces 61 and 62 and extending downward. Specifically, the inclined surfaces 61 and 62 are provided in the central portion of the columnar portions 41 and 42, and the notch portions 46 and 47 are formed below the inclined surfaces 61 and 62. Further, the columnar portion 41 has leg portions 51 and 54 on the side portion of the notch portion 46, and the columnar portion 42 has leg portions 52 and 53 on the side portion of the notch portion 47. By having such leg portions 51, 52, 53, and 54, since the container insertion portion 4 has a sufficient axial length, the container insertion portion 4 is satisfactorily attached to the drug container 2 and an attaching operation is also easier.

Further, in the rubber stopper 1 for the drug container of this embodiment, as illustrated in Figs. 1 to 8, each of the inclined surfaces 61 and 62 has both side portions, and the container insertion portion 4 has the leg portions 51, 52, 53, and 54 provided to sandwich each of the inclined surfaces 61 and 62 and extending downward, on both side portions of the inclined surfaces 61 and 62, and the leg portions 51, 52, 53, and 54 have an outer surface portion 40 extending to at least the lower ends of the inclined surfaces 61 and 62. This allows the lower end of the inclined surface of the rubber stopper to be inserted into the opening portion of the drug container 2 without being caught when the rubber stopper 1 is attached to the drug container 2.

Further, in the rubber stopper 1 for the drug container of this embodiment, as illustrated in Figs. 1 to 8, the legportions 51, 52, 53, and 54 have an outer surface portion 40 which further extends downward beyond the lower ends of the inclined surfaces 61 and 62. This makes attachment of the rubber stopper to the drug container easier.

In the rubber stopper 1 for the drug container of this embodiment, as illustrated in Figs. 1 to 8, the leg portions 51, 52, 53, and 54 have a lower end reduced-diameter portion which reduces in diameter downward from the lower end of the outer surface portion 40. As a result, it is easier to insert the rubber stopper into the drug container.

An axial length from the upper ends to the lower ends of the leg portions 51, 52, 53, and 54 of the container insertion portion 4 is preferably 5 to 10 mm, particularly preferably 6 to 8 mm. Further, an axial length (H) from the upper ends to the lower ends of the inclined surfaces 61 and 62 of the container insertion portion 4 is preferably 2 to 6 mm, particularly preferably 3 to 5 mm. The axial length from the lower ends of the inclined surfaces 61 and 62 to the lower ends of the leg portions 51, 52, 53, and 54 is preferably 1 to 5 mm, particularly preferably 2 to 4 mm.

Further, in the rubber stopper 1 for the drug container of this embodiment, as illustrated in Figs. 1 to 8, the container insertion portion 4 includes two side gap portions 43 and 44 facing each other and communicating with the columnar gap portion 45. Specifically, the two columnar portions 41 and 42 are separated from each other, and the side gap portions 43 and 44 are formed therebetween. In the rubber stopper of this embodiment, the side gap portions 43 and 44 extend from the upper end to the lower end of the container insertion portion 4. Likewise, the columnar gap portion 45 also extends from the upper end to the lower end of the container insertion portion 4. The side gap portions 43 and 44 communicate with the above-described columnar gap portion 45 at the upper portion thereof.

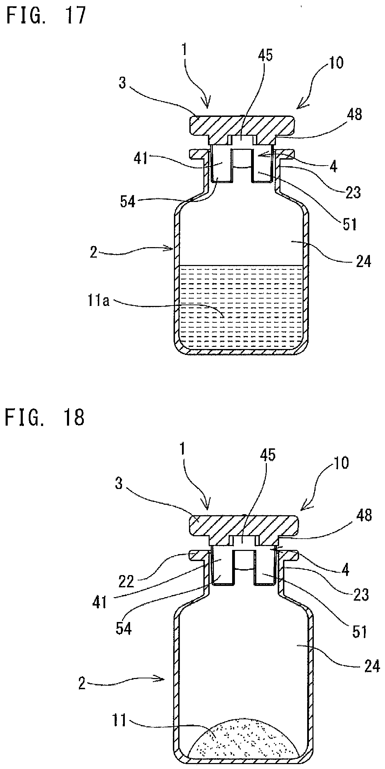

In other words, the side gap portions 43 and 44 and the columnar gap portion 45 are integrated in the upper portion. The width of the side gap portions 43 and 44 is the same as the above-described distance (W) between the facing inner surface portions 63 and 64. Further, the width of the side gap portions 43 and 44 may be wider at the central portion and the lower portion thereof, or the width may be narrower. By providing the columnar gap portion 45 and the side gap portions 43 and 44 communicating therewith, as illustrated in Fig. 17, in a state in which the rubber stopper 1 is temporarily attached to the drug container, the inside and the outside of the container can be allowed to communicate with each other through the gap portion of the rubber stopper.

Further, the four leg portions 51, 52, 53, and 54 extend downward by a predetermined length as described above, and have fan-shaped bottom surface portions 51a, 52a, 53a, and 54a at the lower end thereof. The leg portions 51, 52, 53, and 54 are fan-shaped columnar portions.

Further, the two side gap portions may not extend to the lower end of the container insertion portion 4 but may terminate near the lower portion of the central portion. In this case, the first leg portion and the second leg portion are connected by the connecting portion, and the third leg portion and the fourth leg portion are connected by the connecting portion. Similarly to the leg portions, the connecting portion functions as a guide portion when the rubber stopper is attached to the drug container. In the rubber stopper of this embodiment, the container insertion portion has a crescent-like bottom surface portion on the lower surface.

Further, the rubber stopper 1 for the drug container of this embodiment is provided with inclined surface markers 31 and 32 provided on an upper surface part of the main body 3 at a position above or proximate the inclined surfaces 61 and 62. Specifically, as illustrated in Figs. 1 and 6, the first inclined surface marker 31 is formed at a position just above the inclined surface 61 and slightly from the center of the main body 3. Similarly, a second inclined surface marker 32 is formed at a position just above the inclined surface 62 and slightly from the center of the main body 3. By puncturing the liquid injection needle so that the side hole position of the liquid injection needle coincides with the inclined surface markers 31 and 32, the side hole can be located in the inner surface portions (inner wall surfaces) 63 and 64 of the upper portion of the inclined surfaces 61 and 62.

In this embodiment, each of the markers 31 and 32 is formed by a triangular protruding portion having one vertex directed toward the center of the main body 3. Further, the marker may be a concave portion, a printed portion, or the like, and the shape may be an arrow or the like. Further, the rubber stopper 1 for the drug container of this embodiment is provided with a columnar gap portion marker 33 provided at the upper surface part of the main body 3, which is at a position above the columnar gap portion 45. The columnar gap portion marker 33 is formed by a circular concave portion. The columnar gap portion marker 33 may be a protruding portion, and the shape may be a ring shape, a polygonal shape, or the like.

Further, the drug-container rubber stopper of the present invention is not limited to the above-mentioned ones. For example, as the columnar portions 41 and 42 of the container insertion portion 4, the notch portions 46 and 47 as described above are not provided below the inclined surfaces 61 and 62, and the lower ends of the inclined surfaces 61 and 62 may extend to the lower ends of the columnar portions 41 and 42 of the container insertion portion 4.

Also, only one side gap portion may be included instead of having the two side gap portions 43 and 44 as in the embodiment described above. In addition, it is preferable to provide the inclined surface and the inner wall surface located above the inclined surface at the portion which is not the side gap portion.

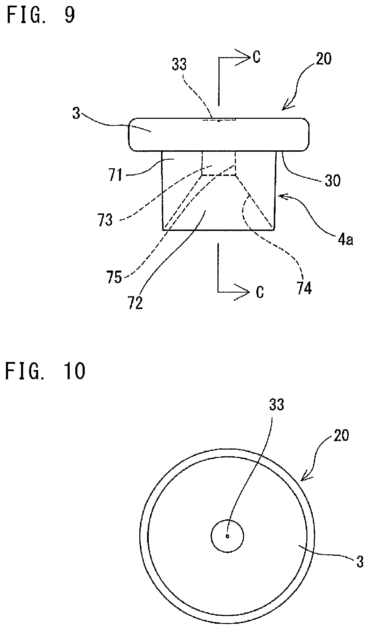

Next, a rubber stopper 20 for a drug container of the embodiment illustrated in Figs. 9 to 12 will be described.

The rubber stopper 20 for the drug container of this embodiment includes a main body 3, and a columnar container insertion portion 4a which extends downward from the lower surface of the main body 3 and can be fitted into the opening portion of the drug container 2 . The container insertion portion 4a has an outer surface portion 40 that makes contact with the inner surface of the opening portion of the drug container 2 when attached to the drug container 2, a columnar gap portion 73 extending downward in a substantially uniform shape downward from the center upper portion of the container insertion portion 4a, an inclined surface 74 extending obliquely downward toward the outer surface portion 40 of the container insertion portion 4a from the columnar gap portion 73, and an inner surface portion 75 extending upward from the upper end of the inclined surface 74. The lower end of the inclined surface 74 reaches or is close to the outer surface portion 40 of the container insertion portion 4a. Further, in the rubber stopper 20 of this embodiment, the inclined surface is a tapered inclined surface 74 which annularly surrounds the columnar gap portion 73 and expands downward.

The main body 3 is in the form of a disc having a predetermined thickness. Further, a lower surface peripheral edge portion 30 of the main body 3 constitutes an annular contact portion which makes contact with the upper surface of an opening portion 22 of the drug container 2 to be described later. The main body 3 is preferably in the shape of a disc. However, as long as the main body 3 has a shape having a peripheral edge portion which is disposed on the radially outer side of the container insertion portion 4a and abuts on the upper surface of the opening portion 22 when attached to the drug container 2, the main body 3 may not have a disc shape. For example, the main body 3 may have a polygonal plate shape having a peripheral edge portion disposed on the radially outer side of the container insertion portion 4a. Further, the lower surface of this peripheral edge portion constitutes the lower surface peripheral edge portion.

The outer surface portion 40 of the container insertion portion 4a makes contact with the inner peripheral surface of the opening portion 22 of the drug container 2 to form a liquid-tight state. The upper portion of the container insertion portion 4 is strongly compressed by the inner peripheral surface of the opening portion 22 of the drug container 2. As the constituent material of the rubber stopper 20, the above-mentioned material is used.

The container insertion portion 4a has an upper columnar portion 71 extending downward from the lower surface of the main body 3 and having a diameter smaller than the main body 3, and a lower columnar portion 72 having a tapered inclined surface 74 extending downward from the upper columnar portion 71 and having an inner surface enlarging in diameter toward the lower end.

The upper columnar portion 71 is formed in a substantially columnar shape and has a columnar gap portion 73 inside. An inner surface portion 75 extending upward from the upper end of the inclined surface 74 is formed by the columnar gap portion 73. In this embodiment, the columnar gap portion 73 has a cylindrical shape. Therefore, the inner surface portion 75 also has a cylindrical shape. The columnar gap portion 73 may have a prismatic shape, an elliptical columnar shape, or the like. Further, the columnar gap portion preferably extends in the uniform shape, but may be slightly larger downward.

An axial length (height) of the inner surface portion 75 of the upper columnar portion 71, in other words, the axial length from the upper end to the lower end of the columnar gap portion 73 is preferably 1 to 8 mm, particularly preferably 3 to 5 mm. The inner diameter of the columnar gap portion 73 is preferably 1.6 to 7 mm, particularly preferably 2.0 to 5.5 mm.

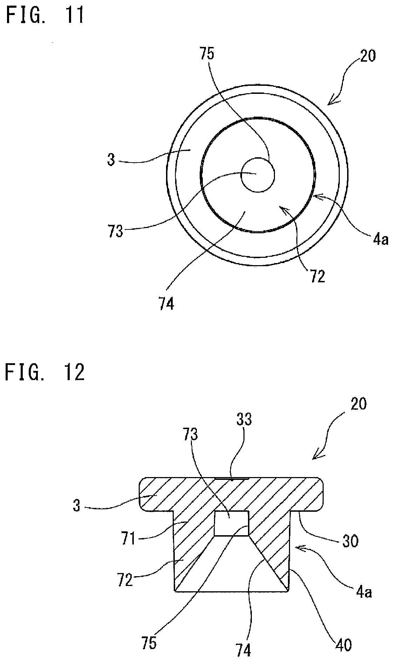

The lower columnar portion 72 has a tapered inclined surface 74 extending downward from the lower end of the columnar gap portion 73 and expanding in diameter toward the lower end. Further, the lower end of the tapered inclined surface 74 reaches or is close to the outer surface portion 40 of the container insertion portion 4a. In this embodiment, as illustrated in Fig. 12, a junction between the lower end of the inclined surface 74 and the outer surface portion 40 is integrated, and the junction has a chamfered and rounded shape. Further, it is preferable that the diameter of the lower columnar portion 72 is slightly reduced downward.

An axial length (height) of the lower columnar portion 72, in other words, the axial length from the upper end to the lower end of the tapered inclined surface 74 is preferably 3 to 8 mm, particularly preferably 4 to 6 mm. An inclination angle of the inclined surface 74 (an inclination angle with respect to the central axis of the container insertion portion 4) is preferably 30 to 50 degrees, particularly preferably 35 to 45 degrees.

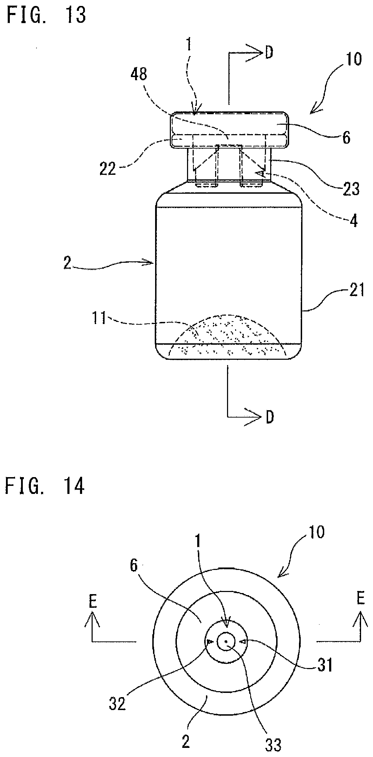

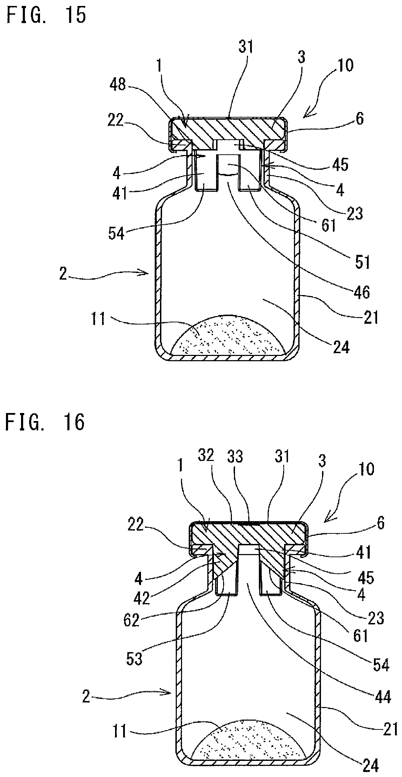

Next, the drug-accommodating drug container 10 of the present invention illustrated in Figs. 13 to 16 will be described.

The drug-accommodating drug container 10 of the present invention includes a drug container 2 having an opening portion, the above-described rubber stopper 1 for the drug container which is mounted in the opening portion 22 of the drug container 2 and seals the opening portion 22, and a drug 11 accommodated in the drug container 2.

The drug container 2 has a cylindrical body portion 21 closed at the lower end thereof and accommodating a drug therein, and a cylindrical neck portion 23 extending upward from the upper end of the body portion 21 and having an opening portion at the upper end thereof, and an inner diameter of the neck portion 23 is smaller than an inner diameter of the body portion 21. Further, the drug container 2 has an opening portion, and is capable of accommodating the drug 11 in an inner portion 24. As the drug container, a rigid or semi-rigid synthetic resin container, a glass container, or the like is used. The drug container 2 has a cylindrical main body (body portion) 21 with a closed lower end, an opening portion 22 having a thick flange, and a neck portion 23 having a smaller diameter than that of another part formed between the opening portion 22 and the main body (body portion) 21. Further, the neck portion 23 from the opening portion 22 of the drug container 2 serves as an accommodating portion for accommodating the container insertion portion 4 of the rubber stopper 1 extending at the same inner diameter. Further, the upper inner surface of the neck portion 23 and the upper end portion 48 of the outer surface portion 40 of the rubber stopper 1 annularly and liquid-tightly come into contact with each other.

As the rubber stopper 1, it is possible to use the rubber stopper of all the above-mentioned embodiments.

The drug 11 is not particularly limited, and examples thereof include platinum-based anticancer agents, protein preparations such as insulin, nitroglycerin, isosorbide nitrate, nicardipine hydrochloride, monoammonium glycyrrhizinate, protein preparation, antitumor agent, vitamin agent (multivitamin), various amino acids, antithrombotic agents such as heparin, antibiotics, analgesics, cardiotonic agent, intravenous anesthetics, antiparkinsonian agent, ulcer therapeutic agent, adrenocortical hormone agent, and antiarrhythmic agent.

In addition, the formulation of the drug is also not particularly limited, and it may be any kind of medical liquid, powdered drug, freeze-dried drug, solid drug, and the like. And, as the drug 11, in a case where using the drug which is dissolved or diluted with a liquid and is easy to foam at the time of dissolution or dilution, the rubber stopper of the present invention is effective. Examples of such drugs include hormone preparations, antibody preparations, and antitumor agents.

In addition, the drug-accommodating drug container 10 of this embodiment is provided with a cover member 6 that covers the peripheral edge portion of the opening portion 22 of the drug container 2 to which the rubber stopper 1 is attached and the peripheral edge portion of the rubber stopper 1. The cover member 6 is preferably made of aluminum, a heat-shrinkable film, or the like, and is preferably in close contact with the rubber stopper and the drug container main body. In the drug-accommodating drug container 10 of this embodiment, the cover member 6 has an opening portion for exposing the inclined surface markers 31 and 32 and the columnar gap portion marker 33. Further, the cover member 6 may cover the entire upper surface of the rubber stopper 1 as long as the inclined surface markers 31 and 32 can be checked and a puncture needle for liquid injection can be punctured. In the drug-accommodating drug container 10 of this embodiment, the cover member 6 has an annular portion and a thin donut-shaped upper surface portion, and the lower end portion of the annular portion covers the annular lower surface of the opening portion 22 of the main body (body portion) 21 of the drug container 2. Further, as the drug container 10, the inside of the drug container 10 may be decompressed.

Further, in the drug-accommodating drug container 10 of the present invention, it is possible to easily store the freeze-dried drug inside. Specifically, as illustrated in Fig. 17, the rubber stopper 1 is temporarily attached to the drug container 2 so that the inner portion 24 and the outside of the drug container 2 communicates with each other via the side gap portions 43 and 44 of the rubber stopper 1 and the columnar gap portion 45 after injecting the medical liquid 11a into the drug container 2.

Specifically, the rubber stopper 1 is inserted into the drug container 2 from the upper side, the portion in which the leg portions 51, 52, 53, and 54 and the inclined surfaces 61 and 62 are formed is accommodated in the drug container, and the upper portions of the container insertion portion 4 (the columnar portions 41 and 42) and the upper portions of the side gap portions 43 and 44 are exposed from the drug container. In this state, by being placed in a freeze dryer and freeze-dried, the medical liquid 11a becomes the freeze-drying agent 11 as illustrated in Fig. 18. When the rubber stopper 1 is pushed in, the upper inner surface of the neck portion 23 and the upper end portion 48 of the outer surface portion 40 of the rubber stopper 1 annularly and liquid-tightly adhere to each other, and the opening portion 22 of the drug container 2 is sealed with the rubber stopper 1. Further, by covering the rubber stopper 1 and the opening portion of the drug container 2 with the cover member 6, the drug-accommodating drug container 10 is created.

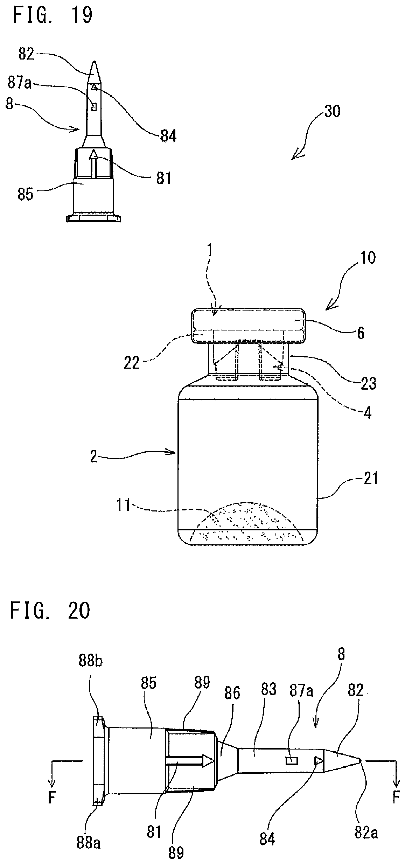

Next, the drug container set 30 of the present invention illustrated in Figs. 19 to 21 will be described.

The drug container set 30 of the present invention includes the drug-accommodating drug container 10 of the present invention and the liquid injection needle 8 capable of penetrating into the rubber stopper 1 of the drug-accommodating drug container 10.

As the liquid injection needle 8, a needle having a blocked puncture end 82a and liquid discharging side holes 87a and 87b provided on the side portion of the front end portion is used.

The liquid injection needle 8 of this embodiment has a puncture portion 82 at a distal end, and includes a hub portion 85 that can be attached to a Luer-taper nozzle of the medical device at a proximal end. The puncture portion 82 reduces in diameter in a tapered shape toward the distal end, and the distal end thereof is closed and has a solid puncture end 82a. The liquid injection needle 8 is provided with a hollow portion 83 which is continuous with the puncture portion 82 and extends toward the proximal end side. The hollow portion 83 has a lumen 80 communicating with the inside of the hub portion 85 and side holes 87a and 87b through which the lumen 80 communicates with the outside.

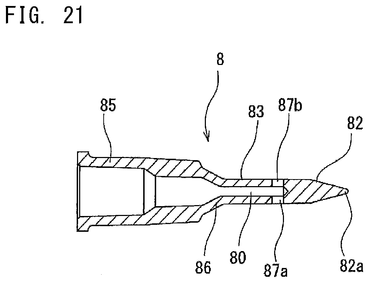

As illustrated in Figs. 20 and 21, the liquid injection needle 8 of this embodiment has a hub portion 85 having a larger diameter than the hollow portion 83 and a predetermined length, and a short tapered portion 86 provided between the hub portion 85 and the hollow portion 83. The hollow portion 83 extends from the proximal end of the puncture portion 82 by a predetermined length, and has a lumen 80 inside. Further, in this embodiment, the hollow portion 83 extends to the distal end of the tapered portion 86. At the distal end portion of the hollow portion 83, side holes 87a and 87b are provided through which the inside of the liquid injection needle 8, in other words, the lumen 80 communicates with the outside. Further, the puncture portion 82 is reduced in diameter toward the distal end in a tapered shape, and has a puncture end 82a at the distal end thereof. The puncture portion 82 is connectable to the rubber stopper 1 of the drug-accommodating drug container 10 by puncturing.

More specifically, as illustrated in Figs. 20 and 21, the liquid injection needle 8 of this embodiment has the solid tapered puncture portion 82, the hollow portion 83, the tapered portion 86, and the hub portion 85 from the distal end side. Further, in the liquid injection needle 8 of this embodiment, the distal end portion of the lumen 80 is reduced in diameter toward the distal end and terminates without reaching the puncture portion 82. The side holes 87a and 87b are provided to communicate with the lumen 80 at the distal end portion of the lumen 80. The puncture portion 82 has a puncture end 82a at the distal end thereof and is a solid puncture portion expanding in diameter at a sharp tapered angle (a tapered divergence angle) toward the proximal end side.

Further, in the liquid injection needle 8 of this embodiment, the length from the distal end of the tapered portion 86 (the proximal end of the hollow portion 83) to the distal end of the side holes 87a and 87b is longer than the thickness of the main body 3 of the upper portion of the columnar gap portion 45 of the rubber stopper 1, and is shorter than the sum of the thickness of the main body and the axial lengths of the inner surface portions 63 and 64 formed by the columnar gap portions 45 (in other words, the distance from the upper surface of the main outer portion 3 to the lower ends of the inner walls 63 and 64).

In the liquid injection needle 8 of this embodiment, the side holes 87a and 87b are located in the formation portions of the inner surface portions 63 and 64 formed by the columnar gap portion 45 in a state of being inserted into the rubber stopper up to the proximal end (the distal end of the tapered portion 86) of the hollow portion 83. Further, the side holes 87a and 87b face the inner surface portions 63 and 64. Therefore, the liquid discharged from the side holes 87a and 87b reliably comes into contact with the inner surface portions 63 and 64. In the liquid injection needle 8 of this embodiment, two side holes are provided to face each other. Only one side hole may be provided.

In addition, a difference between the distance (W) between the facing inner surface portions 63 and 64 and the outer diameter at the side holes 87a and 87b of the liquid injection needle 8 is preferably 0.1 to 3.6 mm, and particularly, preferably 1.0 to 2.5 mm. Accordingly, the liquid discharged from the side holes 87a and 87b can be more reliably brought into contact with the inner surface portions 63 and 64.

It is preferable that the liquid injection needle 8 has a stopper for regulating the penetration length into the rubber stopper. In the liquid injection needle 8 of this embodiment, the tapered portion 86 for connecting the proximal end of the hollow portion 83 and the distal end of the hub portion 85 rapidly expands in diameter subsequently to the hollow portion 83, and puncturing into the rubber stopper 1 is not substantially possible. In other words, the tapered portion 86 functions as a stopper at the time of puncturing.

The hub portion 85 is a cylindrical portion that extends rearward from the proximal end of the tapered portion 86, and has an inner surface shape that can be attached to the Luer-taper nozzle of the medical device. Specifically, the inside of the hub portion 85 is provided with a 6% taper and allows connection of a medical female-type connection portion of a typical standard having a 6% taper (for example, a nozzle portion of a syringe). The medical female-type connection portion of the typical standard is defined as "conical fittings with a 6% (about 3.43°) taper for syringes, needles, and certain other medical device-Part 1: general requirements in ISO 594-1:1986". Two ribs 88a and 88b provided to face each other are provided on the outer surface of the proximal end portion of the hub portion 85. The ribs 88a and 88b can be used as screwed portions when screwing with a screwing portion of another medical device.

Furthermore, the liquid injection needle 8 of this embodiment is provided with a side hole marker 81 provided on the side surface of the liquid injection needle 8 and on the axis of the liquid injection needle passing through the side holes 87a and 87b. Specifically, the side hole marker 81 extending in the axial direction provided on the outer surface near the distal end of the hub portion 85 is provided, and the side holes 87a and 87b are located in front of the extension line of the marker 81. Further, in the liquid injection needle 8 of this embodiment, a front side side hole marker 84 is also provided in front of the side holes 87a and 87b. Further, in the liquid injection needle 8 of this embodiment, a protruding portion 89 extending in the axial direction is provided on the outer surface of the hub portion 85. The protruding portion 89 can be used as a gripping portion of the liquid injection needle 8 and facilitates screwing of the hub portion 85 with other medical devices.

As a material for forming the liquid injection needle 8, for example, thermoplastic resins such as polypropylene, polyethylene, polystyrene, polyamide, polycarbonate, polyvinyl chloride, poly-(4-methylpentene-1), an acrylic resin, an acrylonitrile-butadiene-styrene copolymer, polyester such as polyethylene terephthalate, cyclic olefin resin, polyether ether ketone, polybutylene terephthalate, polyacetal, modified polyphenylene ether, a polyester resin, fluorine resin, polysulfone, polyether imide, polyether sulfone, polyether ketone, polyether lactone, and liquid crystal polyester, or thermosetting resins such as an epoxy resin, an unsaturated polyester resin, a phenol resin, a urea resin, a melamine resin, and a polyurethane resin, and the like can be used.

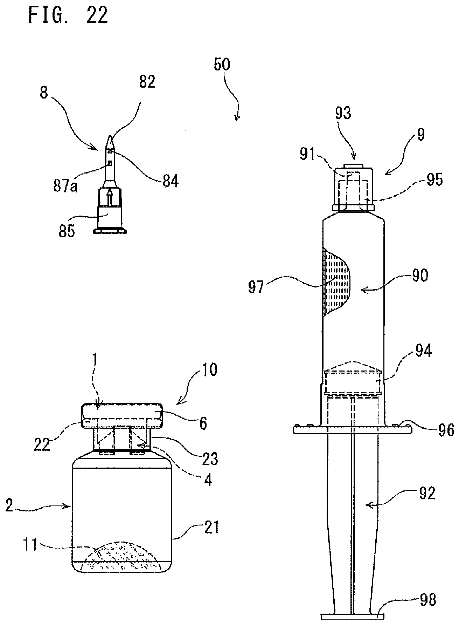

Next, a drug container set 50 of the present invention illustrated in Fig. 22 will be described.

The drug container set 50 of the present invention includes the aforementioned drug-accommodating drug container 10 of the present invention, the liquid injection needle 8 capable of piercing into the rubber stopper 1 of the drug-accommodating drug container 10, and a pre-filled syringe 9. The pre-filled syringe 9 is added to the above-described drug container set 30.

The drug-accommodating drug container 10 and the liquid injection needle 8 are as described above.

As the pre-filled syringe 9, a known one is used. The pre-filled syringe 9 used in this embodiment includes an outer cylinder 90 having a cylindrical outer cylinder main body and a Luer-taper nozzle 91 that is provided at the distal end portion of the outer cylinder main body and has a distal end opening portion, a gasket 94 slidably accommodated inside the outer cylinder 90, a medical liquid 97 filled in the outer cylinder 90, a sealing member 93 for sealing the distal end opening, and a plunger 92 attached to or attachable to a proximal end of the gasket 94.

As the medical liquid 97 to be filled in the pre-filled syringe 9, a drug solution capable of dissolving or diluting the drug in the drug container, for example, water for injection, physiological saline, aqueous glucose solution, or the like is suitable. Incidentally, the medical liquid may contain drugs (for example, vitamins, minerals, cyclosporine, a benzodiazepine-based drug, a sodium-chloride injection solution, antibiotics, and a protein preparation) and can dilute or dissolve the drug.

The pre-filled syringe 9 includes the sealing member 93 (more specifically, a detachable cap) attached to the Luer-taper nozzle 91 so as to seal the distal end opening of the outer cylinder 90. The sealing member may be a peelable film-shaped member.

The outer cylinder 90 is a cylindrical body formed of a transparent or translucent material and, and if necessary, formed of a material having small oxygen permeability and water vapor permeability. The outer cylinder 90 includes the main body, the Luer-taper nozzle 91, a collar 95 which covers the Luer-taper nozzle 91 and has a female screw portion on the inner surface, and a flange 96 provided at the rear end portion. The Luer-taper nozzle 91 is a medical female-type connection portion of a typical standard. The medical female-type connection portion of the typical standard is defined as "conical fittings with a 6% (about 3.43°) taper for syringes, needles, and certain other medical device-Part 1: general requirements in ISO 594-1:1986".

As illustrated in Fig. 22, the gasket 94 includes a main body extending with substantially the same outer diameter and a plurality of annular ribs (two pieces in this embodiment) provided in the main body and slides, while liquid-tightly coming into contact with the inner surface of the outer cylinder 90. The plunger 92 is connected to a rear end portion of the gasket 94. The plunger 92 may be attached in use. The plunger 92 has a pressing portion 98 at a rear end thereof.

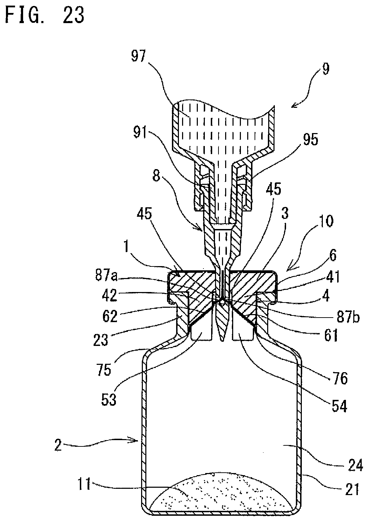

Next, the operation of the drug-container rubber stopper, the drug-accommodating drug container, and the drug container set of the present invention will be described with reference to Fig. 23.

As illustrated in Fig. 23, the liquid injection needle 8 is fitted into the Luer-taper nozzle 91 of the syringe 9 filled with the medical liquid 97, using the hub portion 85. As illustrated in Fig. 23, the liquid injection needle 8 to which the syringe 9 is connected is punctured into the rubber stopper 1 of the drug-accommodating drug container 10. At the time of puncturing, the puncturing is performed, while checking that the side hole marker 84 of the liquid injection needle 8 matches the inclined surface markers 31 and 32 formed on the upper surface of the main body 3 of the rubber stopper 1. By puncturing the liquid injection needle 8 to which the syringe 9 is connected in this way, as illustrated in Fig. 23, the side holes 87a and 87b of the liquid injection needle 8 are located in the columnar gap portion 45, and face the inner surface portions 63 and 64 located on the upper portions of the inclined surfaces 61 and 62.

Further, in this state, by pushing the plunger 92, as illustrated by thick lines 75 and 76 in Fig. 23, the liquid 97 in the pre-filled syringe 9 flows in the direction from the side holes 87a and 87b to the inner surface portions 63 and 64 of the rubber stopper 1, comes into contact with the inner surface portions 63 and 64, then flows downward along the inclined surfaces 61 and 62, further passes through the lower inner surface of the neck portion of the drug container 2 from the inclined surfaces 61 and 62, and flows downward. Therefore, foaming caused by the flow-down of the liquid discharged from the liquid injection needle 8 is less likely to occur.

### Industrial Applicability

A drug-container rubber stopper of the present invention is as follows.
(1) A drug-container rubber stopper configured to be attached to a drug container that comprises: a cylindrical body portion including a closed lower end and accommodating a drug therein; and a cylindrical neck portion extending upward from an upper end of the body portion and including an opening portion formed at an upper end, an inner diameter of the neck portion being smaller than an inner diameter of the body portion and being substantially constant, the drug-container rubber stopper comprising: a main body; and a cylindrical container insertion portion extending downward from a lower surface of the main body and configured to fit into the neck portion of the drug container, wherein the main body includes a peripheral edge portion that is disposed on a radially outer side of the container insertion portion and that is configured to abut on an upper surface of the opening portion when attached to the drug container, wherein the container insertion portion comprises; an outer surface portion that is configured to come into contact with an inner surface of the neck portion of the drug container when attached to the drug container; an upper inner surface portion that forms a columnar gap portion extending downward in a substantially uniform shape from a central portion of the lower surface of the main body; and an inclined surface extending obliquely downward toward the outer surface portion of the container insertion portion from a lower end of the upper inner surface portion, at least the upper end portion of the outer surface portion being configured to annularly and liquid-tightly come into contact with the inner surface of the neck portion, and a lower end of the inclined surface reaches or is close to the outer surface portion of the container insertion portion.
   In the rubber stopper of the present invention, by discharging the medical liquid in a state in which the side hole of the hollow needle is located in the columnar gap portion, the discharged liquid flows along the inner surface of the columnar gap portion and the inclined surface connected to the inner surface and having a lower end reaching or being close to the outer surface of the container insertion portion, and then the liquid flows downward along the inner surface of the drug container. Thus, an occurrence of foaming at the time of liquid discharge is small.
   In addition, the above embodiment may be as follows.
(2) The drug-container rubber stopper according to (1), wherein the inclined surface comprises two inclined surfaces that face each other over a center axis of the columnar gap portion.
(3) The drug-container rubber stopper according to (1) or (2), wherein the container insertion portion has a side gap portion communicating with the columnar gap portion below the upper end portion.
(4) The drug-container rubber stopper according to (3), wherein two side gap portions are disposed to face each other with the columnar gap portion interposed therebetween.
(5) The drug-container rubber stopper according to any one of (1) to (4), wherein the inclined surface includes both side portions, and the container insertion portion includes leg portions disposed on the both side portions of the inclined surface and extending downward, and the leg portions include the outer surface portion extending to at least the lower end of the inclined surface.
(6) The drug-container rubber stopper according to (5), wherein the leg portions include the outer surface portion extending downward from the lower end of the inclined surface.
(7) The drug-container rubber stopper according to (6), wherein the leg portions include a lower end reduced-diameter portion that reduces in diameter downward from the lower end of the outer surface portion.
(8) The drug-container rubber stopper according to any one of (1) to (7), further including: an inclined surface marker disposed on an upper surface of the main body at a position above or proximate the inclined surface.
(9) The drug-container rubber stopper according to the above (1), wherein the inclined surface is a tapered inclined surface that annularly surrounds the columnar gap portion and expands in diameter in a downward direction.
(10) The drug-container rubber stopper according to any one of (1) to (9), further including: a columnar gap portion marker disposed on an upper surface of the main body at a position above the columnar gap portion.
(11) The drug-container rubber stopper according to any one of (1) to (10), wherein the main body is a disc-shaped.
   In addition, a drug-accommodating drug container of the present invention is as follows.
(12) A drug-accommodating drug container including: the drug container; the drug-container rubber stopper according to any one of (1) to (11), which is attached to the drug container and seals the opening portion; and a drug accommodated in the drug container.
   In addition, the above embodiment may be as follows.
(13) The drug-accommodating drug container according to (12), further including: a cover member that covers a peripheral edge portion of the opening portion of the drug container to which the rubber stopper is attached, and that covers a peripheral edge portion of the rubber stopper.
   Further, a drug container set of the present invention is as follows.
(14) A drug container set including: the drug-accommodating drug container according to (12) or (13); and a liquid injection needle configured to pierce into the rubber stopper of the drug-accommodating drug container, wherein the liquid injection needle includes a closed puncture end and a liquid discharging side hole disposed on a side portion of a front end portion thereof.
   In addition, the above embodiment may be as follows.
(15) The drug container set according to (14), wherein the liquid injection needle includes two side holes that face each other.
(16) The drug container set according to (15), wherein the liquid injection needle includes a side hole marker disposed on a side surface of the liquid injection needle and on an axis of the liquid injection needle passing through the side hole.
(17) The drug container set according to any one of (14) to (16), further including: a pre-filled syringe configured to connect to the liquid injection needle and, the pre-filled syringe being filled with a medical liquid.

## Claims

1. A drug-container rubber stopper configured to be attached to a drug container that comprises: a cylindrical body portion including a closed lower end and accommodating a drug therein; and a cylindrical neck portion extending upward from an upper end of the body portion and including an opening portion formed at an upper end thereof, an inner diameter of the neck portion being smaller than an inner diameter of the body portion and being substantially constant, the drug-container rubber stopper comprising:
a main body; and
a cylindrical container insertion portion extending downward from a lower surface of the main body and configured to fit into the neck portion of the drug container,
wherein the main body includes a peripheral edge portion that is disposed on a radially outer side of the container insertion portion and that is configured to abut on an upper surface of the opening portion when attached to the drug container,
wherein, the container insertion portion comprises: an outer surface portion that is configured to come into contact with an inner surface of the neck portion of the drug container when attached to the drug container; an upper inner surface portion that forms a columnar gap portion extending downward in a substantially uniform shape from a central portion of the lower surface of the main body; and an inclined surface extending obliquely downward toward the outer surface portion of the container insertion portion from a lower end of the upper inner surface portion, at least an upper end portion of the outer surface portion being configured to annularly and liquid-tightly come into contact with the inner surface of the neck portion, and a lower end of the inclined surface reaches or is close to the outer surface portion of the container insertion portion.

2. The drug-container rubber stopper according to claim 1, wherein the inclined surface comprises two inclined surfaces that face each other over a center axis of the columnar gap portion.

3. The drug-container rubber stopper according to claim 1 or 2, wherein the container insertion portion includes a side gap portion communicating with the columnar gap portion below the upper end portion.

4. The drug-container rubber stopper according to claim 3, wherein two side gap portions are disposed to face each other with the columnar gap portion interposed therebetween.

5. The drug-container rubber stopper according to any one of claims 1 to 4, wherein the inclined surface includes both side portions, the container insertion portion includes leg portions disposed on the both side portions of the inclined surface and extending downward, and the leg portions include the outer surface portion extending to at least the lower end of the inclined surface.

6. The drug-container rubber stopper according to claim 5, wherein the leg portions include the outer surface portion extending downward from the lower end of the inclined surface.

7. The drug-container rubber stopper according to claim 6, wherein the leg portions include a lower end reduced-diameter portion that reduces in diameter downward from the lower end of the outer surface portion.

8. The drug-container rubber stopper according to any one of claims 1 to 7, further comprising:
an inclined surface marker disposed on an upper surface of the main body at a position above or proximate the inclined surface.

9. The drug-container rubber stopper according to claim 1, wherein the inclined surface is a tapered inclined surface that annularly surrounds the columnar gap portion and expands in diameter in a downward direction.

10. The drug-container rubber stopper according to any one of claims 1 to 9, further comprising:
a columnar gap portion marker disposed on an upper surface of the main body at a position above the columnar gap portion.

11. The drug-container rubber stopper according to any one of claims 1 to 10, wherein the main body is a disc-shaped.

12. A drug-accommodating drug container comprising:
the drug container;
the drug-container rubber stopper according to any one of claims 1 to 11, which is attached to the drug container and seals the opening portion; and
a drug accommodated in the drug container.

13. The drug-accommodating drug container according to claim 12, further comprising: a cover member that covers a peripheral edge portion of the opening portion of the drug container to which the rubber stopper is attached, and that covers a peripheral edge portion of the rubber stopper.

14. A drug container set comprising:
the drug-accommodating drug container according to claim 12 or 13; and
a liquid injection needle configured to pierce into the rubber stopper of the drug-accommodating drug container,
wherein the liquid injection needle includes a closed puncture end and a liquid discharging side hole disposed on a side portion of a front end portion thereof.

15. The drug container set according to claim 14, wherein the liquid injection needle includes two side holes that face each other.

16. The drug container set according to claim 15, wherein the liquid injection needle includes a side hole marker disposed on a side surface of the liquid injection needle and on an axis of the liquid injection needle passing through the side hole.

17. The drug container set according to any one of claims 14 to 16, further comprising: a pre-filled syringe configured to connect to the liquid injection needle and, the pre-filled syringe being filled with a medical liquid.
